(19) Europäisches Patentamt
European Patent Office
Office européen des brevets

(11) Veröffentlichungsnummer : **0 336 185 B1**

(12) # EUROPÄISCHE PATENTSCHRIFT

(45) Veröffentlichungstag der Patentschrift :
**02.09.92 Patentblatt 92/36**

(51) Int. Cl.$^5$ : **C07C 255/27,** C07C 253/30

(21) Anmeldenummer : **89104901.7**

(22) Anmeldetag : **18.03.89**

(54) **1-(N-Formylamino)-2,4-dicyanobutan und ein Verfahren zu dessen Herstellung.**

Die Akte enthält technische Angaben, die nach dem Eingang der Anmeldung eingereicht wurden und die nicht in dieserPatentschrift enthalten sind.

(30) Priorität : **31.03.88 DE 3810973**

(43) Veröffentlichungstag der Anmeldung :
**11.10.89 Patentblatt 89/41**

(45) Bekanntmachung des Hinweises auf die Patenterteilung :
**02.09.92 Patentblatt 92/36**

(84) Benannte Vertragsstaaten :
**CH DE FR GB IT LI NL**

(56) Entgegenhaltungen :
**EP-A- 0 205 131**
**CHEMICAL ABSTRACTS, Band 85, 1976, Seite 252, Zusammenfassung Nr. 16116h, Columbus, Ohio, US; R. SEKURA et al.:"Alpha-aminomethylglutarate, a beta-amino acid analog of glutamate that interacts with glutamine synthetase and the enzymes thatcatalyze glutathione synthesis"**

(73) Patentinhaber : **BAYER AG**
**W-5090 Leverkusen 1 Bayerwerk (DE)**

(72) Erfinder : **Scholl, Hans-Joachim, Dr.**
**Am Feldrain 5**
**W-5000 Köln 80 (DE)**

EP 0 336 185 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft die neue chemische Verbindung 1-(N-Formylamino)-2,4-dicyanobutan und ein Verfahren zu deren Herstellung aus 2.4-Dicyan-1-buten und Formamid.

Es wurde die neue chemische Verbindung 1-(N-Formylamino)-2,4-dicyanobutan gefunden, die der Formel (I) entspricht

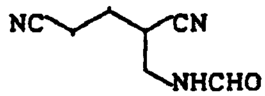
(I).

Es wurde weiterhin ein Verfahren zur Herstellung von 1-(N-Formylamino)-2,4-dicyanobutan gefunden, das dadurch gekennzeichnet ist, daß man 2.4-Dicyan-1-buten und Formamid unter Zusatz eines 4-Aminopyridin-Derivates umsetzt.

Bezogen auf 1 Mol 2.4-Dicyan-1-buten können beispielsweise 0,5 bis 10 Mol Formamid eingesetzt werden. Vorzugsweise beträgt die Menge 1 bis 6 Mol.

Als 4-Aminopyridin-Derivate kommen beispielsweise solche der Formel (II) in Frage

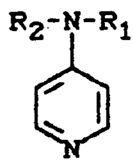
(II)

in der
$R_1$ und $R_2$ unabhängig voneinander für einen einbindigen $C_1$- bis $C_6$-Alkylrest oder $R_1$ und $R_2$ gemeinsam für einen zweibindigen $C_3$- bis $C_5$-Alkylrest stehen.

Bevorzugt wird N,N-Dimethyl-4-aminopyridin oder 4-Pyrrolidinopyridin eingesetzt.

Die Einsatzmenge dieser katalytisch wirkenden Base ist nicht kritisch. Zur Erzielung eines hinreichend schnellen Umsatzes werden üblicherweise 1 bis 12 Mol-% pro Mol 2.4-Dicyan-1-buten verwendet.

Das Verfahren wird vorzugsweise lösungsmittelfrei durchgeführt.

Geeignete Reaktionstemperaturen sind beispielsweise solche im Bereich von 0 bis 120°C, vorzugsweise im Bereich von 20 bis 100° C.

Das Arbeiten bei Normaldruck ist bevorzugt, jedoch kann auch unter erhöhtem Druck gearbeitet werden.

Im übrigen erfordert das erfindungsgemäße Verfahren keine verfahrenstechnischen Besonderheiten. Das gleiche gilt für die Aufarbeitung. Nach Abtrennung der Siederanteile, Formamid und des Katalysators (z.B. durch Destillation oder Dünnschichten) fällt 1-(N-Formylamino)-2,4-dicyanobutan als schwach gefärbte Flüssigkeit an, die normalerweise von üblicher Zwischenproduktreinheit ist, so daß auf weitere Reinigungsprozeduren verzichtet werden kann.

1-(N-Formylamino)-2,4-dicyanobutan ist ein wichtiges Zwischenprodukt. Es eröffnet z.B. einen vorteilhaften Zugang zur Herstellung von α-Aminomethyl-Glutarsäure.

Gemäß EP-A 0 205 131 wird Formamid mit Acrylnitril in Gegenwart tertiärer Stickstoffbasen zu 2-(N-Formylamino)-propionitril umgesetzt.

SEKURA, HOCHREITER, MEISTER, J. Biol. Chem. 251, 2263-2270 (1976) beschreiben die Herstellung von α-Aminomethylglutarsäure durch Hydrolyse von α-Aminomethyl-glutaronitril, allerdings mit sehr geringer Ausbeute,

Beispiel 1

In eine Lösung aus 106 g 2,4-Dicyan-1-buten und 90 g Formamid werden bei Raumtemperatur unter Rühren 7,4 g N,N-Dimethyl-4-aminopyridin eingetragen. Die klare Lösung wird 23 Stunden bei 60°C gerührt und unter Dünnschichtbedingungen (145°C/0,1 mbar) von restlichen Ausgangsmaterialien und Katalysatoranteilen getrennt. Die so erhaltene hellgelbe, klare Sumpfflüssigkeit besteht zu 95 % aus 1-(N-Formylamino)-2,4-dicyano-butan (I) (GC-Analyse).
Ausbeute: 75 g (47 %)
Selektivität: 95 %

| Analyse (%): | C | H | N |
|---|---|---|---|
| gefunden: | 56,0 | 6,0 | 27,4 |
| Theorie | 55,6 | 6,0 | 27,8 |

(bezogen auf $C_7H_9N_3O$)

Beispiel 2

Gemäß Beispiel 1 wird eine Lösung von 430 g 2,4-Dicyan-1-buten, 548 g Formamid und 14,9 g N,N-Dimethyl-4-aminopyridin zur Reaktion gebracht und entsprechend aufgearbeitet.

Man erhält 204 g 1-(N-Formylamino)-2,4-dicyanobutan (I) von 98 % Reinheit (GC-Analyse) und 780 g Destillat.

Ausbeute: 204 g (32 %)
Selektivität: 97 %

| Analyse (%): | C | H | N |
|---|---|---|---|
| gefunden: | 55,8 | 6,1 | 27,5 |
| Theorie: | 55,6 | 6,0 | 27,8 |

## Patentansprüche

1. 1-(N-Formylamino)-2,4-dicyanbutan der Formel

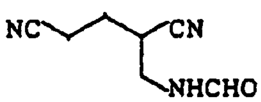

(I).

2. Verfahren zur Herstellung von 1-(N-Formylamino)-2,4-dicyanbutan, dadurch gekennzeichnet, daß man 2.4-Dicyan-1-buten und Formamid unter Zusatz eines 4-Aminopyridin-Derivates umsetzt.

3. Verfahren nach Anspruch 2, dadurch gekennzeichnet, daß man ein 4-Aminopyridin-Derivat der Formel

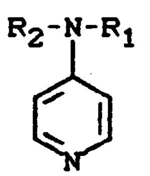

(II)

einsetzt, in der
$R_1$ und $R_2$ unabhängig voneinander für einen einbindigen $C_1$- bis $C_6$-Alkylrest oder $R_1$ und $R_2$ gemeinsam für einen zweibindigen $C_3$- bis $C_5$-Alkylrest stehen.

4. Verfahren nach einem der Ansprüche 2 oder 3, dadurch gekennzeichnet, daß bezogen auf 2.4-Dicyan-1-buten 1 bis 12 mol-% eines 4-Aminopyridin-Derivates zum Einsatz kommen.

## Claims

1. 1-(N-formylamino)-2,4-dicyanobutane corresponding to the following formula

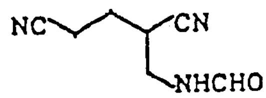

(I).

2. A process for the production of 1-(N-formulamino)-2,4-dicyanobutane, characterized in that 2,4-dicyano-1-butene and formamide are reacted in the presence of a 4-aminopyridine derivative.

3. A process as claimed in claim 2, characterized in that a 4-aminopyridine derivative corresponding to the following formula

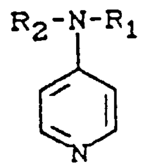

(II)

in which
$R_1$ and $R_2$ independently of one another represent a single-bonded $C_{1-6}$ alkyl radical or $R_1$ and $R_2$ together represent a double-bonded $C_{3-5}$ alkyl radical, is used.

4. A process as claimed in claim 2 or 3, characterized in that the 4-aminopyridine derivative is used in a quantity of 1 to 12 mol-%, based on 2,4-dicyano-1-butene.

## Revendications

1. 1-(N-formylamino)-2,4-dicyanobutane de formule

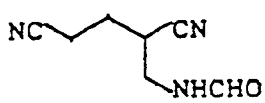

(I).

2. Procédé de production de 1-(N-formylamino)-2,4-dicyanobutane, caractérisé en ce qu'on fait réagir du 2,4-dicyano-1-butène et du formamide avec addition d'un dérivé de 4-aminopyridine.

3. Procédé suivant la revendication 2, caractérisé en ce qu'on utilise un dérivé de 4-aminopyridine de formule

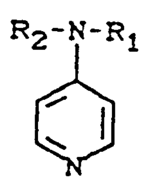

(II)

dans laquelle
$R_1$ et $R_2$ représentent, indépendamment l'un de l'autre, un reste alkyle en $C_1$ à $C_6$ à une seule liaison ou bien $R_1$ et $R_2$ forment conjointement un reste alkyle en $C_3$ à $C_5$ à deux liaisons.

4. Procédé suivant l'une des revendications 2 et 3, caractérisé en ce qu'on utilise, par rapport au 2,4-dicyano-1-butène, 1 à 12 moles % d'un dérivé de 4-aminopyridine.